(19) Europäisches Patentamt
European Patent Office
Office européen des brevets

(11) EP 3 908 396 B1

(12) EUROPEAN PATENT SPECIFICATION

(45) Date of publication and mention of the grant of the patent:
**28.02.2024 Bulletin 2024/09**

(21) Application number: **20700078.7**

(22) Date of filing: **09.01.2020**

(51) International Patent Classification (IPC):
***B01D 67/00*** $^{(2006.01)}$ ***B01D 69/02*** $^{(2006.01)}$
***B01D 69/08*** $^{(2006.01)}$ ***B01D 69/14*** $^{(2006.01)}$
***B01D 71/28*** $^{(2006.01)}$ ***B01D 71/68*** $^{(2006.01)}$
***A61L 33/00*** $^{(2006.01)}$ ***A61M 1/16*** $^{(2006.01)}$
***A61M 1/36*** $^{(2006.01)}$

(52) Cooperative Patent Classification (CPC):
**B01D 69/08; A61M 1/3672; A61M 1/3673; B01D 67/00111; B01D 67/0093; B01D 69/02; B01D 69/144; B01D 71/28; B01D 71/441; B01D 71/68;** B01D 2313/68; B01D 2323/36; B01D 2323/38; B01D 2325/16; B01D 2325/34

(86) International application number:
**PCT/EP2020/050346**

(87) International publication number:
**WO 2020/144244 (16.07.2020 Gazette 2020/29)**

# (54) MEMBRANE WITH IMMOBILIZED ANTICOAGULANT AND PROCESS FOR PRODUCING SAME

MEMBRAN MIT IMMOBILISIERTEM ANTICOAGULANS UND VERFAHREN ZU IHRER HERSTELLUNG

MEMBRANE AVEC ANTICOAGULANT IMMOBILISÉ ET SON PROCÉDÉ DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.01.2019 EP 19151083**

(43) Date of publication of application:
**17.11.2021 Bulletin 2021/46**

(73) Proprietor: **Gambro Lundia AB**
**226 43 Lund (SE)**

(72) Inventors:
- **GEBERT, Michael**
  **72411 Bodelshausen (DE)**
- **HULKO, Michael**
  **71149 Bondorf (DE)**
- **MENDA, Ralf**
  **89250 Senden (DE)**

(74) Representative: **Perchenek, Nils**
**Gambro Dialysatoren GmbH**
**Legal and Intellectual Property**
**Holger Crafoord-Strasse 26**
**72379 Hechingen (DE)**

(56) References cited:
**EP-A1- 3 431 171**
**JP-A- 2001 276 215**
**US-A- 5 840 190**
**US-A1- 2003 021 826**
**US-A1- 2018 238 871**

- **Zubakova Lb ET AL: "[Interaction of poly-2-methyl-5-vinyl-N-ethylpyridinium bromide and heparin studied by means of ultraviolet and infrared spectroscopy]. - PubMed - NCBI", , 1 January 1977 (1977-01-01), XP055616351, Retrieved from the Internet: URL:https://www.ncbi.nlm.nih.gov/pubmed/614540?dopt=Abstract [retrieved on 2019-08-29]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 908 396 B1

## Description

### Technical Field

The present disclosure relates to an anticoagulant-coated microporous hollow fiber membrane showing reduced thrombogenicity. The disclosure further relates to a method for producing the membrane and a filtration and/or diffusion device comprising the membrane.

### Background of the Invention

In extracorporeal blood circuits, systemic anticoagulation is generally used to prevent the coagulation of blood, i.e., the formation of microthrombi and blood clotting. Heparin is the most commonly used anticoagulant. Systemic anticoagulation with heparin is associated with increased bleeding risk and heparin is quite expensive. Therefore, membranes having the ability to immobilize heparin are highly desirable, as they reduce or even eliminate the need for systemic doses of heparin.

US 2003/0021826 A1 proposes binding an anticoagulation agent in a stable manner to the surface of semipermeable support membranes essentially comprised of a copolymer of acrylonitrile and at least one anionic or anionizable monomer. The anticoagulation agent can exert its anticoagulating activity without being leached out into the blood or plasma during treatment by extracorporeal circulation and to reduce the quantity of anticoagulation agent used systemically in the patient during an extracorporeal blood treatment session. The surface of the semipermeable support membrane intended to be brought into contact with the blood or plasma is coated in succession with a cationic polymer carrying cationic groups which can form an ionic bond with anionic or anionizable groups of polyacrylonitrile, for example, polyethyleneimine (PEI), and an anticoagulation agent carrying anionic groups capable of forming an ionic bond with cationic groups of said cationic polymer (for example, heparin).

WO 2004/056459 A1 discloses a permselective asymmetric membrane suitable for hemodialysis, comprising at least one hydrophobic polymer, e.g. polyethersulfone, and at least one hydrophilic polymer, e.g. polyvinylpyrrolidone. The outer surface of the hollow fiber membrane has pore openings in the range of 0.5 to 3 um and the number of pores in the outer surface is in the range of 10,000 to 150,000 pores per $mm^2$.

International patent application PCT/EP2018/069458 discloses positively charged membranes prepared from a spinning solution comprising polyethersulfone, polyvinylpyrrolidone, and a polymer bearing ammonium groups. The positively charged membranes retain endotoxins by adsorptive binding.

US 5 840 190 A discloses a surface modified biocompatible membrane. The membrane is comprised of polysulfone, polyvinylpyrrolidone and polyethyleneimine (PEI). The surface of the membrane is modified by reacting it with a bioactive compound and a coupling agent. In the working examples, heparin partly degraded by nitrous acid is covalently coupled to the membrane surface using sodium cyanoborohydride as coupling agent.

It now has been found that anticoagulants like heparin can be immobilized on positively charged membranes prepared from a spinning solution comprising polyethersulfone, polyvinylpyrrolidone, and a polymer bearing ammonium groups, selected from polyvinylpyridines bearing ammonium groups and copolymers of vinylpyridine and styrene bearing ammonium groups. The resulting heparin-coated microporous hollow fiber membranes show reduced thrombogenicity. Filtration and/or diffusion devices comprising the heparin-coated membranes reduce or even eliminate the need for systemic anticoagulation in extracorporeal blood circuits.

Surprisingly, it has been found that the ability of the membranes of the present disclosure to immobilize heparin is higher than that of membranes comprising other constituents bearing localized positive charges.

### Summary of the Invention

The present disclosure provides a porous hollow fiber membrane having an anticoagulant immobilized thereon. The membrane comprises polysulfone, polyethersulfone, or polyarylethersulfone; polyvinylpyrrolidone; and a polymer bearing ammonium groups which is selected from polyvinylpyridines bearing ammonium groups and copolymers of vinylpyridine and styrene bearing ammonium groups. The present disclosure also provides a process for producing the porous hollow fiber membrane having an anticoagulant immobilized thereon. The present disclosure further provides filtration and/or diffusion devices comprising the porous hollow fiber membrane having an anticoagulant immobilized thereon. The filtration and/or diffusion devices, e.g., hemodialyzers, can be used in the extracorporeal treatment of blood, e.g., in hemodialysis, hemodiafiltration, and hemofiltration.

### Detailed Description of the Invention

In one aspect of the present invention, a porous hollow fiber membrane having an anticoagulant immobilized

thereon is provided. The membrane comprises a blend of i) polysulfone (PSU), polyethersulfone (PESU), or polyarylethersulfone (PAES); ii) polyvinylpyrrolidone (PVP), and iii) at least one polymer bearing ammonium groups.

The at least one polymer bearing ammonium groups is selected from polyvinylpyridines bearing ammonium groups and copolymers of vinylpyridine and styrene bearing ammonium groups. In one embodiment, the ammonium groups are quaternary ammonium groups, e.g., N-alkylpyridinium groups.

Suitable counter ions for the ammonium groups include chloride, bromide, sulfate, hydrogen sulfate, monomethylsulfate, trifluoromethane sulfonate, carbonate, hydrogen carbonate, phosphate, hydrogen phosphate, dihydrogen phosphate, acetate, lactate, and citrate. In one embodiment, the counter ion is chloride. In another embodiment, the counter ion is bromide. In still another embodiment, the counter ion is sulfate.

In one embodiment, a polymer bearing quaternary ammonium groups is obtained by reacting a polyvinylpyridine or a copolymer of vinylpyridine and styrene with an alkylating agent, e.g., an alkyl sulfate like dimethyl sulfate or diethyl sulfate. In one embodiment, 1 to 20 mol%, e.g., 2 to 10 mol%, or 3 to 8 mol% of the pyridine groups in the polyvinylpyridine are N-alkylated. In a particular embodiment, 20 mol% of the pyridine groups in the polyvinylpyridine are N-alkylated. In another particular embodiment, 5 mol% of the pyridine groups in the polyvinylpyridine are N-alkylated. In one embodiment, the counter ion of the N-alkylpyridinium groups is monomethylsulfate. In one embodiment, the polymer bearing quaternary ammonium groups has a weight average molecular weight in the range of from 10 to 500 kDa, e.g., 150 to 200 kDa.

In one embodiment, the polymer bearing quaternary ammonium groups corresponds to the formula

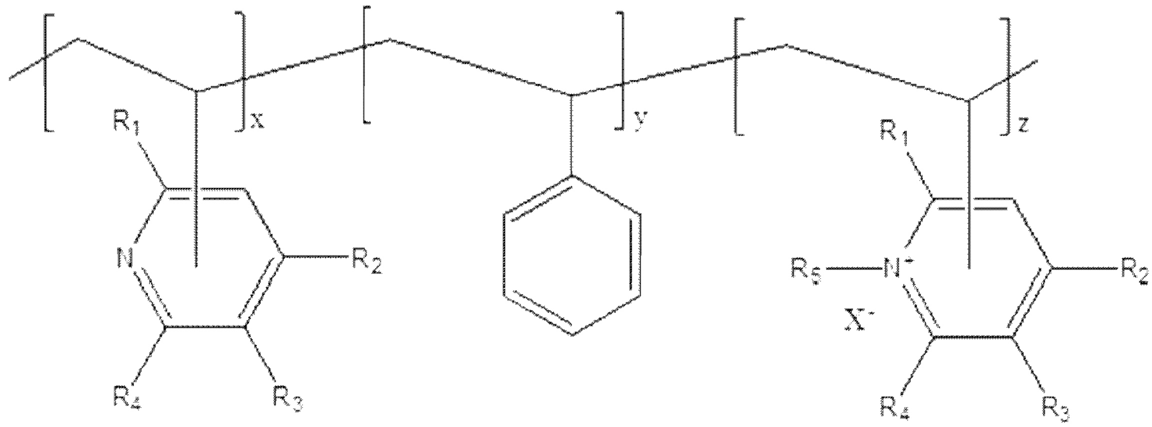

wherein

| | |
|---|---|
| $R_1$, $R_2$, $R_3$, $R_4$ | are individually selected from H, alkyl, benzyl; |
| $R_5$ | is selected from alkyl, benzyl; |
| $X^-$ | is selected from $Cl^-$, $Br^-$, $SO_4^{2-}$, $CH_3SO_4^-$; |

with

$$0 \leq x \leq 1;$$

$$0 \leq y \leq 0.5;$$

$$0 \leq z \leq 0.5;$$

and

$$x + y + z = 1.$$

Examples of suitable polyethersulfones include polyethersulfones having a weight average molecular weight (determined by GPC) of about 40,000 to 100,000 Da. In one embodiment, a polyethersulfone having a weight average molecular weight $M_w$ in the range of from 50 to 60 kDa is used. An example is a polyethersulfone having a weight average

molecular weight $M_w$ of 58 kDa (determined by GPC) and a polydispersity $M_w/M_n$ of 3.3. In another embodiment, a polyethersulfone having a weight average molecular weight $M_w$ in the range of from 65 to 80 kDa is used. An example is a polyethersulfone having a weight average molecular weight $M_w$ of 75 kDa (determined by GPC) and a polydispersity $M_w/M_n$ of 3.4. In yet another embodiment, a polyethersulfone having a weight average molecular weight $M_w$ in the range of from 80 to 100 kDa is used. An example is a polyethersulfone having a weight average molecular weight $M_w$ of 92 kDa (determined by GPC) and a polydispersity $M_w/M_n$ of 3.0.

Examples of suitable polysulfones include polysulfones having a weight average molecular weight (determined by GPC) of about 40,000 to 80,000 Da. In one embodiment, a polysulfone having a weight average molecular weight $M_w$ in the range of from 45 to 65 kDa is used. An example is a polysulfone having a weight average molecular weight $M_w$ of 52 kDa (determined by GPC) and a polydispersity $M_w/M_n$ of 3.5. Another example is a polysulfone having a weight average molecular weight $M_w$ of 60 kDa (determined by GPC) and a polydispersity $M_w/M_n$ of 3.7.

Suitable polyvinylpyrrolidones include homopolymers of vinylpyrrolidone having a weight average molecular weight in the range of from 50 kDa to 2,000 kDa. These homopolymers generally have a number average molecular weight in the range of from 14 kDa to 375 kDa. Examples of suitable polyvinylpyrrolidones for preparing the membranes of the invention are Luvitec® K30, Luvitec® K85, Luvitec® K90, and Luvitec® K90HM, respectively, all available from BASF SE.

In one embodiment of the invention, the polyvinylpyrrolidone comprised in the porous hollow fiber membrane consists of a high (≥ 100 kDa) and a low (< 100 kDa) weight average molecular weight component.

An example of a suitable polyvinylpyrrolidone having a weight average molecular weight <100 kDa is a polyvinylpyrrolidone having a weight average molecular weight of 50 kDa and a number average molecular weight of 14 kDa. Such a product is available from BASF SE under the trade name Luvitec® K30.

Examples of suitable polyvinylpyrrolidones having a weight average molecular weight >100 kDa include poylvinylpyrrolidones having a weight average molecular weight in the range of about 1,000 to 2,000 kDa, e.g., 1,100 to 1,400 kDa, or 1,400 to 1,800 kDa; a number average molecular weight of about 200 to 400 kDa, e.g., 250 to 325 kDa, or 325 to 325 kDa; and a polydispersity $M_w/M_n$ of about 4 to 5, for instance, 4.3 to 4.4, or 4.3 to 4.8.

One embodiment of the invention uses a polyvinylpyrrolidone homopolymer having a weight average molecular weight of about 1,100 kDa; and a number average molecular weight of about 250 kDa.

Another embodiment of the invention uses a polyvinylpyrrolidone homopolymer having a weight average molecular weight of about 1,400 kDa; and a number average molecular weight of about 325 kDa.

Still another embodiment of the invention uses a polyvinylpyrrolidone homopolymer having a weight average molecular weight of about 1,800 kDa; and a number average molecular weight of about 375 kDa.

The hollow fiber membranes can have a symmetric wall structure or an asymmetric wall structure. In one embodiment, the membrane wall has a symmetric sponge structure. In another embodiment, the membrane wall has an asymmetric sponge structure, i.e., the size of the pores in the hollow fiber wall increases from the inner surface of the membrane towards the outer surface. In yet another embodiment of the process, the membrane wall has an asymmetric wall structure and comprises a layer having a finger structure, i.e., featuring macrovoids having a volume-equivalent diameter of more than 5 μm.

In one embodiment, the hollow fiber membrane has an inner diameter of from 150 to 250 pm, for instance, from 180 to 250 μm. In another embodiment, the inner diameter is in the range of from 185 μm to 195 μm. In still another embodiment, the inner diameter is in the range of from 210 μm to 220 μm.

In one embodiment, the wall thickness of the hollow fiber membranes is in the range of from 15 μm to 60 μm. In one embodiment, the wall thickness is 33 μm to 37 μm. In another embodiment, the wall thickness is 38 to 42 μm. In still another embodiment, the wall thickness is 43 μm to 47 μm. In yet another embodiment, the wall thickness is 48 μm to 52 μm.

In one embodiment, the membrane has sieving coefficients, measured according to EN 1283 at 37°C in bovine plasma having a protein content of 60 g/l, for vitamin $B_{12}$ of 1.0, for inulin of 1.0, for $\beta_2$-microglobulin of at least 0.7, and for albumin of less than 0.01.

In one embodiment, the membrane has a molecular weight cutoff (MWCO) in whole blood in the range of from 10 kDa to 40 kDa. In a further embodiment, the average pore size in the selective layer of the membrane is in the range of from 2 to 5 nm.

In one embodiment, the hydraulic permeability (Lp) for water of the membrane is in the range of from $1.10^{-4}$ $cm^3$/ ($cm^2$·bar·sec) to $250 \cdot 10^{-4}$ $cm^3$/ ($cm^2$·bar·sec), for example from $70 \cdot 10^{-4}$ $cm^3$/ ($cm^2$ -bar .sec) to $150 \cdot 10^{-4}$ $cm^3$/ ($cm^2$·bar·sec).

The membrane has an anticoagulant grafted onto its surface. The anticoagulant agent forms an ionic bond with the ammonium groups of the polymer. The anticoagulation agent may comprise at least one compound of the glycoaminoglycanes family with anticoagulation activity, and is preferably selected from the group consisting of unfractionated heparin, fractionated heparin, danaparoids, heparin derivatives, and mixtures of said products. The use of unfractionated heparin may prove to be especially beneficial. The surface concentration of the deposited anticoagulation

agent usually is in the range of from 1,000 to 30,000 IU/$m^2$, e.g., in the range of from 1,500 to 10,000 IU/$m^2$.

It is a further object of the present invention to provide a hollow fiber membrane useful for producing a device for the extracorporeal purification of blood, e.g., a dialyzer. The surface area of a dialyzer comprising the hollow fiber membrane of the present disclosure may vary, but will usually be in a range of from 1.0 to 2.3 $m^2$. Dialyzers comprising the membrane of the present disclosure can be assembled as known in the art.

Sterilization of the devices will normally be effected by irradiation with gamma rays or using ETO. In a particular embodiment, radiation dose used is in the range of from 25 to 50 kGy, for instance, 25 kGy. In another embodiment, the device is sterilized with ETO.

The present disclosure also provides a process for making the hollow fiber membrane. The process involves the production of a porous hollow fiber membrane bearing localized positive charges.

In one embodiment, the porous hollow fiber membrane bearing positive charges is prepared by a continuous solvent phase inversion spinning process comprising the steps of

a) dissolving at least one polyethersulfone, at least one polyvinylpyrrolidone, and at least one polymer bearing ammonium groups, in N-methyl-2-pyrrolidone to form a polymer solution;

b) extruding the polymer solution through an outer ring slit of a nozzle with two concentric openings into a precipitation bath; simultaneously

c) extruding a center fluid through the inner opening of the nozzle;

d) washing the membrane obtained; and subsequently

e) drying the membrane;

wherein the polymer solution comprises from 10 to 15 wt%, relative to the total weight of the polymer solution, of polyethersulfone, and from 5 to 10 wt%, relative to the total weight of the polymer solution, of polyvinylpyrrolidone, and from 0.03 to 2 wt%, relative to the total weight of the solution, of at least one polymer bearing ammonium groups, and the polymer bearing ammonium groups is selected from polyvinylpyridines bearing ammonium groups and copolymers of vinylpyridine and styrene bearing ammonium groups.

Suitable polymers bearing ammonium groups include polyvinylpyridines bearing quaternary ammonium groups, e.g., N-alkylpyridinium groups.

Suitable counter ions for the ammonium groups include chloride, bromide, sulfate, monomethylsulfate, hydrogen sulfate, trifluoromethane sulfonate, carbonate, hydrogen carbonate, phosphate, hydrogen phosphate, dihydrogen phosphate, acetate, lactate, and citrate. In one embodiment, the counter ion is chloride. In another embodiment, the counter ion is monomethylsulfate.

In one embodiment, the polymer solution comprises from 0.03 to 2 wt%, e.g., 0.05 to 1 wt%, or 0.1 to 0.5 wt%, relative to the total weight of the solution, of a polymer bearing ammonium groups. In one embodiment, the ammonium groups are quaternary ammonium groups. In one embodiment, the polymer bearing ammonium groups has a number average molecular weight of 50 to 2,000 kDa, e.g., 100 to 250 kDa, for instance, 150 to 200 kDa. In another embodiment, the polymer bearing ammonium groups has a weight average molecular weight of 10 to 500 kDa, e.g., 150 to 200 kDa.

In one embodiment, the polymer bearing ammonium groups is a polyvinylpyridine having a weight average molecular weight of 150 to 200 kDa, wherein 3 to 8 mol% of the pyridine groups in the polyvinylpyridine have been transformed into N-alkylpyridinium groups with sulfate as counter ion.

In still another embodiment, the polymer bearing ammonium groups is a polyvinylpyridine having a weight average molecular weight of 150 to 200 kDa, wherein 18 to 22 mol% of the pyridine groups in the polyvinylpyridine have been transformed into N-alkylpyridinium groups with sulfate as counter ion.

The concentration of polyethersulfone in the polymer solution generally is in the range of from 10 to 15 wt%, for instance, 12 to 14 wt%.

In one embodiment, the polymer solution comprises a polyethersulfone having a weight average molecular weight $M_w$ in the range of from 90 to 95 kDa is used. An example is a polyethersulfone having a weight average molecular weight $M_w$ of 92 kDa and a polydispersity $M_w/M_n$ of 3. In another embodiment, polymer solution comprises a polyethersulfone having a weight average molecular weight $M_w$ in the range of from 70 to 80 kDa is used. An example is a polyethersulfone having a weight average molecular weight $M_w$ of 75 kDa and a polydispersity $M_w/M_n$ of 3.4.

The concentration of polyvinylpyrrolidone in the polymer solution generally is in the range of from 5 to 10 wt%, e.g., from 6 to 8 wt%.

In one embodiment of the process, the polymer solution comprises a high ($\geq$ 100 kDa) and a low (< 100 kDa) molecular weight PVP. In one embodiment, 50-60 wt%, e.g., 50-55 wt%, based on the total weight of PVP in the polymer

solution, is high molecular weight component, and 40-60 wt%, e.g., 45-50 wt%, based on the total weight of PVP in the polymer solution, is low molecular weight component.

In one embodiment, the polymer solution comprises 4 to 6 wt% of a polyvinylpyrrolidone having a weight average molecular weight of 50 kDa; and 1 to 3 wt% of a polyvinylpyrrolidone having a weight average molecular weight of 1,100 kDa.

In one embodiment, the polymer solution comprises from 2 to 5 wt%, e.g., 3 wt%, relative to the total weight of the solution, of water.

In one embodiment of the process for preparing the membrane, the center fluid comprises 40 to 60 wt% of water and 40 to 60 wt% of NMP, for instance, 50 to 60 wt% of water and 40 to 50 wt% of NMP, or 52 to 55 wt% of water and 45 to 48 wt% of NMP, e.g., 54 wt% of water and 46 wt% of NMP, relative to the total weight of the center fluid.

In one embodiment of the process, the precipitation bath is comprised of water. In one embodiment of the process, the precipitation bath has a temperature in the range of from 10 to 30°C, for instance, 15 to 25°.

In one embodiment of the process for preparing the membrane, the temperature of the spinneret is in the range of from 50 to 60°C, e.g., 55-58°C.

In one embodiment of the process, the distance between the opening of the nozzle and the precipitation bath is in the range of from 10 to 120 cm, e.g., 90 to 110 cm.

In one embodiment of the process, the spinning speed is in the range of from 20 to 80 m/min, e.g., 30 to 50 m/min.

The membrane then is washed to remove residual solvent and low molecular weight components. In a particular embodiment of a continuous process for producing the membrane, the membrane is guided through several water baths. In certain embodiments of the process, the individual water baths have different temperatures. For instance, each water bath may have a higher temperature than the preceding water bath.

Subsequently, an anticoagulant is grafted onto at least one surface of the membrane. In one embodiment, the surface is the lumen surface of the membrane, i.e., its interior wall surface. In another embodiment, the surface is the outer surface of the membrane, i.e., its exterior wall surface. In still another embodiment, both the inner wall surface and the surface of the pore channels within the membrane are grafted with the anticoagulant. In yet another embodiment, both the outer wall surface and the surface of the pore channels within the membrane are grafted with the anticoagulant. In still another embodiment, the inner wall surface, the pore channels within the membrane, and the outer wall surface of the membrane are grafted with the anticoagulant.

The anticoagulant forms an ionic bond with the ammonium groups present on the surface of the membrane. The anticoagulant is attached to the membrane surface by electrostatic interaction between the negatively charged anticoagulant and the positively charged membrane surface. In one embodiment, the anticoagulant comprises at least one member of the glycoaminoglycane family having anticoagulation activity. In a further embodiment, the anticoagulant is selected from the group consisting of unfractionated heparin, fractionated heparin, danaparoids, heparin derivatives, and mixtures of said products. In one embodiment, the anticoagulant is unfractionated heparin. In one embodiment, the concentration of the anticoagulation agent on the membrane surface after the grafting step is in the range of from 1,000 to 30,000 IU/$m^2$, e.g., in the range of from 1,500 to 10,000 IU/$m^2$.

The grafting can be performed by contacting a solution of the anticoagulant with the membrane surface. In one embodiment, the membrane is flushed with an aqueous solution of the anticoagulant and subsequently rinsed with water or saline to remove excess anticoagulant. In another embodiment, the membrane is flushed with an aqueous solution of the anticoagulant and subsequently dried to evaporate the solvent. In still another embodiment, the membrane is soaked with an aqueous solution of the anticoagulant making use of the capillary force generated by the hollow fiber.

The grafting can be performed before or after the membrane has been incorporated into a diffusion and/or filtration device. In other words, the grafting can also be performed on a diffusion and/or filtration device comprising the porous hollow fiber membrane bearing positive charges of the present disclosure. For instance, a diffusion and/or filtration device, e.g., a hemodialyzer comprising a bundle of porous hollow fiber membrane bearing positive charges can be flushed with an aqueous solution of the anticoagulant to effect the grafting; and subsequently the device is rinsed with water or saline to remove excess anticoagulant. In another embodiment, the device is flushed with an aqueous solution of the anticoagulant to effect the grafting; and subsequently the membranes in the device are dried and the solvent is evaporated.

After the grafting, the membrane can be post-processed. Examples of post-processing include extensive rinsing of the membrane to remove loosely bound anticoagulant; radiation treatment of the membrane to cross-link the anticoagulant with other polymers present in the membrane; drying of the membrane; cross-linking polymer chains of polymers present in the membrane by heating the membrane.

The present disclosure thus provides a simple process for producing membranes coated with anticoagulants like heparin. An advantage of the membrane of the present disclosure is that no intermediate or priming layer between the base material and the anticoagulant coating is present, and the process for making the membrane does not require the additional steps for forming such a priming layer. The process of the present disclosure also does not involve an additional chemical reaction step for covalently coupling the anticoagulant to the membrane surface and consequently

does not involve the use of further coupling agents. The present disclosure provides a simple, scalable procedure for lowcost manufacturing of heparin-coated membranes.

The subject matter of the present disclosure is further described in the following working examples.

## Methods

### Preparation of mini-modules

Mini-modules [= fibers in a housing] are prepared by cutting the fibers to a length of 20 cm, drying the fibers for 1 h at 40°C and < 100 mbar and subsequently transferring the fibers into the housing. The ends of the fibers are closed using a UV-curable adhesive. The mini-module is dried in a vacuum drying oven at 60 °C over night, and then the ends of the fibers are potted with polyurethane. After the polyurethane has hardened, the ends of the potted membrane bundle are cut to reopen the fibers. The mini-module ensures protection of the fibers.

### Hydraulic Permeability (Lp) of mini-modules

The hydraulic permeability of a mini-module is determined by pressing a defined volume of water under pressure through the mini-module, which has been sealed on one side, and measuring the required time. The hydraulic permeability is calculated from the determined time t, the effective membrane surface area A, the applied pressure p and the volume of water pressed through the membrane V, according to equation (1):

$$Lp = V / [p \cdot A \cdot t] \qquad (1)$$

The effective membrane surface area A is calculated from the fiber length and the inner diameter of the fiber according to equation (2)

$$A = \pi \cdot d_i \cdot l \cdot [cm^2] \qquad (2)$$

with

$d_i$ = inner diameter of fiber [cm]
l = effective fiber length [cm]

The mini-module is wetted thirty minutes before the Lp-test is performed. For this purpose, the mini-module is put in a box containing 500 mL of ultrapure water. After 30 minutes, the mini-module is transferred into the testing system. The testing system consists of a water bath that is maintained at 37°C and a device where the mini-module can be mounted. The filling height of the water bath has to ensure that the minimodule is located underneath the water surface in the designated device.

In order to avoid that a leakage of the membrane leads to a wrong test result, an integrity test of the mini-module and the test system is carried out in advance. The integrity test is performed by pressing air through the mini-module that is closed on one side. Air bubbles indicate a leakage of the mini-module or the test device. It has to be checked if the leakage is due to an incorrect mounting of the mini-module in the test device or if the membrane leaks. The mini-module has to be discarded if a leakage of the membrane is detected. The pressure applied in the integrity test has to be at least the same value as the pressure applied during the determination of the hydraulic permeability in order to ensure that no leakage can occur during the measurement of the hydraulic permeability because the pressure applied is too high.

### Determination of membrane surface charge

The anionic azo dye Acid Orange II

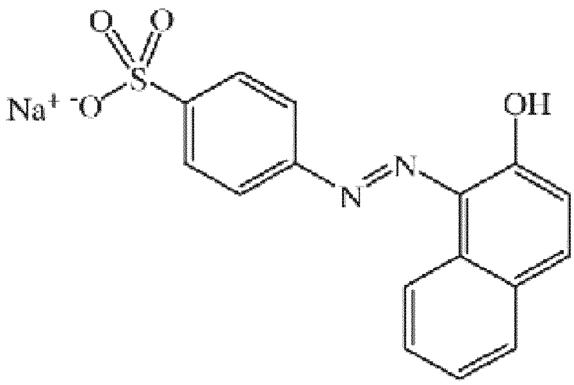

binds to positively charged groups on the membrane surface. To determine the surface charge of the membranes, the minimodules were rinsed filtrating with a solution containing Acid Orange II. The amount of positively charged groups on the inner and outer membrane surfaces was determined by measuring the absorbance of the dye solution before and after passing the minimodule. The more dye bound on the membranes, the higher the positive surface charge. No dye is immobilized in a minimodule containing an uncharged reference membrane. The method is described in Desalination and Water Treatment 57(7) (2016) 3218-3226.

**Heparin coating of hollow fibers in mini-modules**

A solution of heparin (Heparin sodium 194 IU/mg, Celsus Laboratories Inc., Cincinnati, USA) and ultrapure water (Milli-Q® Advantage A10, Merck KGaA, Darmstadt, Germany) with a concentration of 1,136 IU/ml was prepared. The dialysate side of the minimodule was closed to ensure that no filtration from blood side to dialysate side could take place. The blood side of the minimodule was filled completely with heparin solution. After filling, the minimodule was incubated at room temperature for 20 minutes. After coating, the minimodules were rinsed with 200 ml of a 0,9% isotonic saline solution (Fresenius Kabi Deutschland GmbH, Bad Homburg, Germany) using a flow rate of 10 ml/min.

**Quantitative determination of immobilized heparin**

A glycine buffer was prepared from 3,84 mg/l glycine (Merck KGaA, Darmstadt, Germany), 2,8 mg/l NaCl (Merck KGaA, Darmstadt, Germany) and water. The pH was adjusted to 11 ± 0,1 with a 30% NaOH solution (VWR, Darmstadt, Germany). The minimodule was rinsed with the glycine buffer at a flow rate of 2,3 ml/min. After 10 minutes, 1 ml of the collected effluent was sampled. After 20 minutes, the minimodule was emptied completely. From the extract after 20 minutes, a sample was collected as well. Heparin concentration in the samples was determined using an Azure A assay.

Azure A is a blue colored, metachromatic dye which changes the color from blue to purple-red when binding to sulfated polysaccharides such as heparin. This color change is measured photometrically at a wavelength of 630 nm. The heparin concentration was determined based on a standard curve which covered a concentration range from 0 mg/l to 12 mg/l heparin. Control samples with known concentrations of 1,2 mg/l, 6 mg/l and 10,8 mg/l of heparin were measured simultaneously each time. 200 μl of the aqueous Azure A (Dye content ~80%, Sigma-Aldrich Chemie GmbH, Steinheim, Germany) solution with a concentration of 25 mg/l Azure A were added to 100 μl of the standard samples, control samples and samples to be measured, respectively, in microplate wells. After storing the microplate in the dark for 15 minutes, the measurement was performed using a microplate photometer (EL 808 Ultra Microplate Reader, BioTek Instruments Inc., Winooski, USA).

**Determination of hemocompatibility**

The hemocompatibility of the membranes was investigated by the perfusion of minimodules with human blood *in vitro.* First, the minimodule was rinsed with about 400 ml of 0,9 % isotonic saline solution at 37°C. Then a reservoir of 30 ml of human blood was recirculated through the minimodule at a flow rate of 9 ml/min for 120 minutes. Samples having a volume of 900 μl were taken at 0 min, 60 min, 90 min, and 120 min and transferred into micro tubes (Micro Tube 1,5 ml EASY CAP, Sarstedt AG & Co. KG, Nümbrecht, Germany) containing 100 μl of a 10 % trisodium citrate solution to inhibit further coagulation of the sample. The samples were stored on ice. After the experiment, the samples were centrifuged with 2000 x g at 4 °C for 30 minutes. Three aliquots of each sample with 100 μl of the supernatant plasma were frozen at -20 °C.

Determination of human TAT complex

The activation of coagulation due to the contact with foreign surfaces was examined by the quantitative determination of thrombin-antithrombin III complex (TAT) in human plasma by means of a sandwich enzyme-linked immunosorbent assay (ELISA) (Enzygnost® TAT micro, Siemens Healthcare Diagnostics Products GmbH, Germany).
After thawing, the aliquoted plasma samples were diluted with human plasma. The dilution factor was chosen depending on the expected TAT concentration and varied between 1:3 and 1:30. Standard samples (range from 2 to 60 pg/l), control samples and the plasma for dilution were assayed in duplicates; while for the samples single determination was sufficient. 50 μl of diluent and 50 μl of the samples were transferred into a well of a microplate and the TAT complex bonded to the antibodies on the microplate. After a washing process, 100 μl of the second enzyme-conjugated antibody were added and removed by washing after 30 minutes of incubation. An enzymatic reaction which occurs by addition of chromogen was measured photometrically (EL 808 Ultra Microplate Reader, BioTek Instruments Inc., Winooski/USA) at 490 nm within one hour.

## Examples

| | |
|---|---|
| Additive 1: | block copolymer of ethylene oxide and epichlorohydrin, reacted with 40 mol%, in relation to the chloride groups in the copolymer, of 1,4-diazabicyclo[2.2.2]octane; having a number average molecular weight of 150 to 200 kDa; |
| Additive 2: | polyvinylpyridine having a weight average molecular weight of 150 to 200 kDa, comprising 5 mol%, based on the pyridine moieties in the polymer, of N-alkylpyridinium groups; the counter ion is monomethylsulfate; |
| Additive 3: | polyvinylpyridine having a weight average molecular weight of 150 to 200 kDa, comprising 20 mol%, based on the pyridine moieties in the polymer, of N-alkylpyridinium groups; the counter ion is monomethylsulfate; |
| Additive 4: | ethyleneimine homopolymer having a weight average molecular weight of 750 kDa (Lupasol® P, BASF SE) . |

### Comparative Example 1

A solution of 13.5% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); 5.0% w/w PVP having a weight average molecular weight of about 50 kDa (Luvitec® K30, BASF SE); and 0.5% w/w of Additive 1; in 3% w/w water, 2.85% w/w dimethylacetamide and 73.15% w/w NMP was extruded through the outer ring slit of a spinneret with two concentric openings into a coagulation bath containing water. A solution containing 54.5% w/w water and 45.5% w/w NMP was used as the center fluid and extruded through the inner opening of the spinneret. The temperature of the spinneret was 54°C; the temperature of the coagulation bath was 20°C and the air gap 100 cm. The fibers were spun at a speed of 50 m/min.
The fibers subsequently were guided through a sequence of water baths containing demineralized water at temperatures in the range of from 70°C to 85°C. The wet fiber subsequently was dried in an on-line drying step and texturized. The fiber obtained had an inner diameter of 190 μm and a wall thickness of 35 μm.
A mini-module was prepared as described above and hydraulic permeability of the fibers was tested as described above. The mini-module showed a Lp of $70 \cdot 10^{-4}$ $cm^3/(cm^2 \cdot bar \cdot sec)$.
The membranes of a minimodule were coated with heparin as described above and the amount of heparin immobilized on the membranes was determined as described above. After 20 minutes of extraction, the amount of heparin extracted from the minimodule was less than the determination limit of the method.

### Comparative Example 2 (corresponding to US 5 840 190 A)

A solution of 14% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); and 2% w/w of Additive 4; in 2% w/w water and 80% w/w NMP was extruded through the outer ring slit of a spinneret with two concentric openings into a coagulation bath containing water. A solution containing 56% w/w water and 44% w/w NMP was used as the center fluid and extruded through the inner opening of the spinneret. The temperature of the spinneret was 48°C; the temperature of the coagulation bath was 25°C and the air gap 100 cm. The fibers were spun

at a speed of 40 m/min.

The fibers subsequently were guided through a sequence of water baths containing demineralized water at temperatures in the range of from 25°C to 70°C. The wet fiber subsequently was dried in an on-line drying step and texturized. The fiber obtained had an inner diameter of 190 μm and a wall thickness of 35 μm.

A mini-module was prepared as described above and hydraulic permeability of the fibers was tested as described above. The mini-module showed a Lp of $22 \cdot 10^{-4}$ $cm^3/(cm^2 \cdot bar \cdot sec)$.

The membranes of three minimodules were coated with heparin as described above and the amount of heparin immobilized on the membranes was determined as described above. After 20 minutes of extraction, (20±4) IU of heparin were extracted from each minimodule (n=3).

As polyethyleneimine is water-soluble, the additive and the heparin bound to it are leached from the membrane during use in aqueous media, e.g., blood.

**Example 1**

A solution of 13.5% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); 5.0% w/w PVP having a weight average molecular weight of about 50 kDa (Luvitec® K30, BASF SE); and 0.5% w/w of Additive 2; in 3% w/w water and 76% w/w NMP was extruded through the outer ring slit of a spinneret with two concentric openings into a coagulation bath containing water. A solution containing 54% w/w water and 46% w/w NMP was used as the center fluid and extruded through the inner opening of the spinneret. The temperature of the spinneret was 54°C; the temperature of the coagulation bath was 20°C and the air gap 100 cm. The fibers were spun at a speed of 50 m/min.

The fibers subsequently were guided through a sequence of water baths containing demineralized water at temperatures in the range of from 70°C to 85°C. The wet fiber subsequently was dried in an on-line drying step and texturized. The fiber obtained had an inner diameter of 190 μm and a wall thickness of 35 μm. The membrane showed an asymmetric sponge structure.

Three mini-modules were prepared as described above and hydraulic permeability of the fibers was tested as described above. The Lp of the mini-modules was determined to be $(112 \pm 7) \cdot 10^{-4}$ $cm^3/ (cm^2$ -bar .sec) (n=3) .

**Example 2**

A solution of 13.5% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); 5.0% w/w PVP having a weight average molecular weight of about 50 kDa (Luvitec® K30, BASF SE); and 0.5% w/w of Additive 2; in 3% w/w water and 76% w/w NMP was extruded through the outer ring slit of a spinneret with two concentric openings into a coagulation bath containing water. A solution containing 54% w/w water and 46% w/w NMP was used as the center fluid and extruded through the inner opening of the spinneret. The temperature of the spinneret was 58°C; the temperature of the coagulation bath was 20°C and the air gap 100 cm. The fibers were spun at a speed of 50 m/min.

The fibers subsequently were guided through a sequence of water baths containing demineralized water at temperatures in the range of from 70°C to 85°C. The wet fiber subsequently was dried in an on-line drying step and texturized. The fiber obtained had an inner diameter of 190 μm and a wall thickness of 35 μm. The membrane showed an asymmetric sponge structure.

Two mini-modules were prepared as described above and hydraulic permeability of the fibers was tested as described above. The Lp of the mini-modules was determined to be $(120 \pm 11) \cdot 10^{-4}$ $cm^3/(cm^2 \cdot bar \cdot sec)$ (n=2).

**Example 3**

A solution of 13.5% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); 5.0% w/w PVP having a weight average molecular weight of about 50 kDa (Luvitec® K30, BASF SE); and 0.5% w/w of Additive 2; in 3% w/w water and 76% w/w NMP was extruded through the outer ring slit of a spinneret with two concentric openings into a coagulation bath containing water. A solution containing 54% w/w water and 46% w/w NMP was used as the center fluid and extruded through the inner opening of the spinneret. The temperature of the spinneret was 58°C; the temperature of the coagulation bath was 20°C and the air gap 100 cm. The fibers were spun at a speed of 30 m/min.

The fibers subsequently were guided through a sequence of water baths containing demineralized water at temperatures in the range of from 70°C to 85°C. The wet fiber subsequently was dried in an on-line drying step and

texturized. The fiber obtained had an inner diameter of 190 μm and a wall thickness of 35 μm. The membrane showed an asymmetric sponge structure.

Mini-modules were prepared as described above and hydraulic permeability of the fibers was tested on one mini-module as described above. The mini-module showed a Lp of $72 \cdot 10^{-4}$ $cm^3/(cm^2 \cdot bar \cdot sec)$.

Surface charge of the membranes was determined as described above. 1.67 μM of Acid Orange II were immobilized on the membranes of the mini-module.

Hemocompatibility was determined as described above by rinsing a mini-module with human blood and measuring formation of human TAT complex. TAT concentrations for a minimodule without heparin coating were determined to be 40 pg/l at 0 min, 190 pg/l at 60 min, 280 pg/l at 90 min, and 375 pg/l at 120 min.

The membranes of three minimodules were coated with heparin as described above and the amount of heparin immobilized on the membranes was determined as described above. After 20 minutes of extraction, (30±3) IU of heparin were extracted from each minimodule (n=3).

Hemocompatibility of the heparin-coated mini-modules was determined as described above by rinsing a mini-module with human blood and measuring formation of human TAT complex. TAT concentrations for a mini-module with heparin coating were determined to be 40 pg/l at 0 min, 45 pg/l at 60 min, 65 pg/l at 90 min, and 65 pg/l at 120 min.

**Example 4**

A solution of 13.75% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); 5.0% w/w PVP having a weight average molecular weight of about 50 kDa (Luvitec® K30, BASF SE); and 0.25% w/w of Additive 2; in 3% w/w water and 76% w/w NMP was extruded through the outer ring slit of a spinneret with two concentric openings into a coagulation bath containing water. A solution containing 54% w/w water and 46% w/w NMP was used as the center fluid and extruded through the inner opening of the spinneret. The temperature of the spinneret was 58°C; the temperature of the coagulation bath was 20°C and the air gap 100 cm. The fibers were spun at a speed of 30 m/min.

The fibers subsequently were guided through a sequence of water baths containing demineralized water at temperatures in the range of from 70°C to 85°C. The wet fiber subsequently was dried in an on-line drying step and texturized. The fiber obtained had an inner diameter of 190 μm and a wall thickness of 35 μm. The membrane showed an asymmetric sponge structure.

A mini-module was prepared as described above and hydraulic permeability of the fibers was tested as described above. The mini-module showed a Lp of $75 \cdot 10^{-4}$ $cm^3/(cm^2 \cdot bar \cdot sec)$.

**Example 5**

A solution of 13.95% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); 5.0% w/w PVP having a weight average molecular weight of about 50 kDa (Luvitec® K30, BASF SE); and 0.05% w/w of Additive 2; in 3% w/w water and 76% w/w NMP was extruded through the outer ring slit of a spinneret with two concentric openings into a coagulation bath containing water. A solution containing 54% w/w water and 46% w/w NMP was used as the center fluid and extruded through the inner opening of the spinneret. The temperature of the spinneret was 58°C; the temperature of the coagulation bath was 20°C and the air gap 100 cm. The fibers were spun at a speed of 30 m/min.

The fibers subsequently were guided through a sequence of water baths containing demineralized water at temperatures in the range of from 70°C to 85°C. The wet fiber subsequently was dried in an on-line drying step and texturized. The fiber obtained had an inner diameter of 190 μm and a wall thickness of 35 μm. The membrane showed an asymmetric finger structure, i.e., a structure containing a plurality of macrovoids.

Two mini-modules were prepared as described above and hydraulic permeability of the fibers was tested as described above. The Lp of the mini-modules was determined to be $(93 \pm 1) \cdot 10^{-4}$ $cm^3/(cm^2 \cdot bar \cdot sec)$ (n=2) .

Surface charge of the membranes was determined as described above. 0.68 μM of Acid Orange II were immobilized on the membranes of the minimodule.

The membranes of a minimodule were coated with heparin as described above and the amount of heparin immobilized on the membranes was determined as described above. After 20 minutes of extraction, 5.7 IU of heparin were extracted from the minimodule.

**Example 6**

A solution of 13.5% w/w polyethersulfone having a weight average molecular weight of about 75 kDa (Ultrason®

E 6020, BASF SE); 2.0% w/w PVP having a weight average molecular weight of about 1,100 kDa (Luvitec® K85, BASF SE); 5.0% w/w PVP having a weight average molecular weight of about 50 kDa (Luvitec® K30, BASF SE); and 0.5% w/w of Additive 3; in 3% w/w water and 76% w/w NMP was extruded through the outer ring slit of a spinneret with two concentric openings into a coagulation bath containing water. A solution containing 54% w/w water and 46% w/w NMP was used as the center fluid and extruded through the inner opening of the spinneret. The temperature of the spinneret was 58°C; the temperature of the coagulation bath was 20°C and the air gap 100 cm. The fibers were spun at a speed of 30 m/min.

The fibers subsequently were guided through a sequence of water baths containing demineralized water at temperatures in the range of from 70°C to 85°C. The wet fiber subsequently was dried in an on-line drying step and texturized. The fiber obtained had an inner diameter of 190 μm and a wall thickness of 35 μm. The membrane showed an asymmetric sponge structure.

A mini-module was prepared as described above and hydraulic permeability of the fibers was tested as described above. The mini-module showed a Lp of $54 \cdot 10^{-4}$ $cm^3/(cm^2 \cdot bar \cdot sec)$.

Surface charge of the membranes was determined as described above. (3.41±0.06) μM of Acid Orange II were immobilized on the membranes of the minimodule (n=2).

The membranes of three minimodules were coated with heparin as described above and the amount of heparin immobilized on the membranes was determined as described above. After 20 minutes of extraction, (23±7) IU of heparin were extracted from each minimodule (n=3).

Hemocompatibility of the heparin-coated mini-modules was determined as described above by rinsing a mini-module with human blood and measuring formation of human TAT complex. TAT concentrations for a mini-module with1 heparin coating were determined to be 35 pg/l at 0 min, 50 pg/l at 60 min, 80 pg/l at 90 min, and 90 pg/l at 120 min.

**Example 7**

A solution of 14% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); and 0.5% w/w of Additive 2; in 3% w/w water and 80.5% w/w NMP was extruded through the outer ring slit of a spinneret with two concentric openings into a coagulation bath containing water. A solution containing 53% w/w water and 47% w/w NMP was used as the center fluid and extruded through the inner opening of the spinneret. The temperature of the spinneret was 48°C; the temperature of the coagulation bath was 25°C and the air gap 100 cm. The fibers were spun at a speed of 40 m/min.

The fibers subsequently were guided through a sequence of water baths containing demineralized water at temperatures in the range of from 25°C to 70°C. The wet fiber subsequently was dried in an on-line drying step and texturized. The fiber obtained had an inner diameter of 190 μm and a wall thickness of 35 μm. The membrane showed an asymmetric sponge structure.

A mini-module was prepared as described above and hydraulic permeability of the fibers was tested as described above. The mini-module showed a Lp of $238 \cdot 10^{-4}$ $cm^3/(cm^2 \cdot bar \cdot sec)$.

The membranes of three minimodules were coated with heparin as described above and the amount of heparin immobilized on the membranes was determined as described above. After 20 minutes of extraction, (50±2) IU of heparin were extracted from each minimodule (n=3).

**Claims**

1. A porous hollow fiber membrane having an anticoagulant immobilized thereon; the membrane comprising a blend of i) polysulfone, polyethersulfone or polyarylethersulfone; ii) polyvinylpyrrolidone; and iii) at least one polymer bearing ammonium groups selected from the group consisting of polyvinylpyridines bearing ammonium groups, and copolymers of vinylpyridine and styrene bearing ammonium groups.

2. The membrane of claim 1, wherein the ammonium groups are N-alkylpyridinium groups.

3. The membrane of claims 1 or 2, wherein the polymer bearing ammonium groups is the reaction product of a polyvinylpyridine or a copolymer of vinylpyridine and styrene and an alkylating agent.

4. The membrane of any one of claims 1 to 3, wherein from 3 to 8 mol% of the pyridine groups in the polymer bearing ammonium groups are N-alkylated.

5. The membrane of any one of claims 1 to 4, wherein the polymer bearing ammonium groups has a weight average

molecular weight in the range of from 150 to 200 kDa.

6. A process for preparing a porous hollow fiber membrane having an anticoagulant immobilized thereon, comprising the steps of

   a) dissolving at least one polyethersulfone, at least one polyvinylpyrrolidone, and at least one polymer bearing ammonium groups, in N-methyl-2-pyrrolidone to form a polymer solution;
   b) extruding the polymer solution through an outer ring slit of a nozzle with two concentric openings into a precipitation bath; simultaneously
   c) extruding a center fluid through the inner opening of the nozzle;
   d) washing the membrane obtained; and subsequently
   e) grafting an anticoagulant onto at least one surface of the membrane;

   wherein the polymer solution comprises from 10 to 15 wt%, relative to the total weight of the polymer solution, of polyethersulfone, and from 5 to 10 wt%, relative to the total weight of the polymer solution, of polyvinylpyrrolidone, and from 0.03 to 2 wt%, relative to the total weight of the solution, of at least one polymer bearing ammonium groups, the polymer bearing ammonium groups being selected from polyvinylpyridines bearing ammonium groups and copolymers of vinylpyridine and styrene bearing ammonium groups.

7. The process of claim 6, wherein the polymer bearing ammonium groups is the reaction product of i) a polyvinylpyridine or a copolymer of vinylpyridine and styrene; and ii) an alkylating agent.

8. The process of claims 6 or 7, wherein the center fluid comprises 50 to 60 wt% of water and 40 to 50 wt% of NMP, relative to the total weight of the center fluid.

9. The process of any one of claims 6 to 8, wherein the precipitation bath has a temperature in the range of from 15 to 25°C.

10. The process of any one of claims 6 to 9, wherein the anticoagulant is a glycoaminoglycane.

11. The process of claim 10, wherein the anticoagulant is unfractionated heparin.

12. The process of any one of claims 6 to 11, wherein the anticoagulant is grafted onto at least one surface of the membrane by contacting an aqueous solution of the anticoagulant with the membrane surface.

13. A filtration and/or diffusion device comprising a plurality of porous hollow fiber membranes according to any one of claims 1 to 5.

14. The filtration and/or diffusion device of claim 13, wherein the device is a hemodialyzer.

**Patentansprüche**

1. Poröse Hohlfasermembran mit einem darauf immobilisierten Antikoagulans, wobei die Membran eine Mischung aus i) Polysulfon, Polyethersulfon oder Polyarylethersulfon; ii) Polyvinylpyrrolidon; und iii) mindestens einem Ammoniumgruppen tragendes Polymer, ausgewählt aus der Gruppe bestehend aus Ammoniumgruppen tragenden Polyvinylpyridinen und Ammoniumgruppen tragenden Copolymeren aus Vinylpyridin und Styrol enthält.

2. Membran nach Anspruch 1, wobei die Ammoniumgruppen N-Alkylpyridiniumgruppen sind.

3. Membran nach Anspruch 1 oder 2, wobei das Ammoniumgruppen tragende Polymer das Reaktionsprodukt eines Polyvinylpyridins oder eines Copolymers aus Vinylpyridin und Styrol mit einem Alkylierungsmittel ist.

4. Membran nach einem der Ansprüche 1 bis 3, wobei 3 bis 8 Mol-% der Pyridingruppen im Ammoniumgruppen tragenden Polymer N-alkyliert sind.

5. Membran nach einem der Ansprüche 1 bis 4, wobei das Ammoniumgruppen tragende Polymer ein gewichtsmittleres Molekulargewicht im Bereich von 150 bis 200 kDa aufweist.

6. Verfahren zur Herstellung einer porösen Hohlfasermembran mit einem darauf immobilisierten Antikoagulans, umfassend die Schritte:

a) Lösen mindestens eines Polyethersulfons, mindestens eines Polyvinylpyrrolidons und mindestens eines Ammoniumgruppen tragenden Polymers in N-Methyl-2-pyrrolidon unter Bildung einer Polymerlösung;
b) Extrudieren der Polymerlösung durch einen äußeren Ringschlitz einer Düse mit zwei konzentrischen Öffnungen in ein Fällbad; gleichzeitig
c) Extrudieren einer Zentralflüssigkeit durch die innere Öffnung der Düse;
d) Waschen der erhaltenen Membran; und anschließend
e) Aufpfropfen eines Antikoagulans auf mindestens eine Oberfläche der Membran;

wobei die Polymerlösung 10 bis 15 Gew.-%, bezogen auf das Gesamtgewicht der Polymerlösung, Polyethersulfon und 5 bis 10 Gew.-%, bezogen auf das Gesamtgewicht der Polymerlösung, Polyvinylpyrrolidon enthält, und 0,03 bis 2 Gew.-%, bezogen auf das Gesamtgewicht der Lösung, mindestens eines Ammoniumgruppen tragenden Polymers, wobei das Ammoniumgruppen tragende Polymer aus Ammoniumgruppen tragenden Polyvinylpyridinen und Ammoniumgruppen tragenden Copolymeren aus Vinylpyridin und Styrol ausgewählt ist.

7. Verfahren nach Anspruch 6, wobei das Ammoniumgruppen tragende Polymer das Reaktionsprodukt i) eines Polyvinylpyridins oder eines Copolymeren aus Vinylpyridin und Styrol; und ii) eines Alkylierungsmittels ist.

8. Verfahren nach Anspruch 6 oder 7, wobei die Zentralflüssigkeit 50 bis 60 Gew.-% Wasser und 40 bis 50 Gew.-% NMP enthält, bezogen auf das Gesamtgewicht der Zentralflüssigkeit.

9. Verfahren nach einem der Ansprüche 6 bis 8, wobei das Fällbad eine Temperatur im Bereich von 15 bis 25°C aufweist.

10. Verfahren nach einem der Ansprüche 6 bis 9, wobei das Antikoagulans ein Glykosaminoglykan ist.

11. Verfahren nach Anspruch 10, wobei das Antikoagulans unfraktioniertes Heparin ist.

12. Verfahren nach einem der Ansprüche 6 bis 11, wobei das Antikoagulans auf mindestens eine Oberfläche der Membran aufgepfropft wird, indem eine wässrige Lösung des Antikoagulans mit der Membranoberfläche in Kontakt gebracht wird.

13. Filtrations- und/oder Diffusionsvorrichtung, die mehrere poröse Hohlfasermembranen nach einem der Ansprüche 1 bis 5 umfasst.

14. Die Filtrations- und/oder Diffusionsvorrichtung nach Anspruch 13, wobei die Vorrichtung ein Hämodialysator ist.

## Revendications

1. Membrane poreuse à fibres creuses sur laquelle est immobilisé un anticoagulant ; la membrane comprenant un mélange de i) polysulfone, polyéthersulfone ou polyaryléthersulfone ; ii) polyvinylpyrrolidone ; et iii) au moins un polymère portant des groupes ammonium choisi dans le groupe constitué des polyvinylpyridines portant des groupes ammonium, et des copolymères de vinylpyridine et de styrène portant des groupes ammonium.

2. Membrane selon la revendication 1, dans laquelle les groupes ammonium sont des groupes N-alkylpyridinium.

3. Membrane selon les revendications 1 ou 2, dans laquelle le polymère portant des groupes ammonium est le produit de réaction d'une polyvinylpyridine ou d'un copolymère de vinylpyridine et de styrène avec d'un agent alkylant.

4. Membrane selon l'une quelconque des revendications 1 à 3, dans laquelle de 3 à 8 % en moles des groupes pyridine dans le polymère portant des groupes ammonium sont N-alkylés.

5. Membrane selon l'une quelconque des revendications 1 à 4, dans laquelle le polymère portant des groupes ammonium a un poids moléculaire moyen en poids compris entre 150 et 200 kDa.

6. Procédé de préparation d'une membrane poreuse à fibres creuses sur laquelle est immobilisé un anticoagulant,

comprenant les étapes:

a) dissoudre au moins une polyéthersulfone, au moins une polyvinylpyrrolidone et au moins un polymère portant des groupes ammonium dans de la N-méthyl-2-pyrrolidone pour former une solution polymère ;
b) extruder la solution de polymère à travers une fente annulaire extérieure d'une buse comportant deux ouvertures concentriques dans un bain de précipitation ; simultanément
c) extruder un fluide central à travers l'ouverture interne de la buse ;
d) laver la membrane obtenue ; et par suite
e) greffer un anticoagulant sur au moins une surface de la membrane ;

dans lequel la solution de polymère comprend de 10 à 15 % en poids, par rapport au poids total de la solution de polymère, de polyéthersulfone, et de 5 à 10 % en poids, par rapport au poids total de la solution de polymère, de polyvinylpyrrolidone, et de 0,03 à 2 % en poids, par rapport au poids total de la solution, d'au moins un polymère portant des groupes ammonium, le polymère portant des groupes ammonium étant choisi parmi les polyvinylpyridines portant des groupes ammonium et les copolymères de vinylpyridine et de styrène portant des groupes ammonium.

**7.** Procédé selon la revendication 6, dans lequel le polymère portant des groupes ammonium est le produit de réaction de i) une polyvinylpyridine ou un copolymère de vinylpyridine et de styrène ; avec ii) un agent alkylant.

**8.** Procédé selon les revendications 6 ou 7, dans lequel le fluide central comprend 50 à 60 % en poids d'eau et 40 à 50 % en poids de NMP, par rapport au poids total du fluide central.

**9.** Procédé selon l'une quelconque des revendications 6 à 8, dans lequel le bain de précipitation a une température comprise dans la plage de 15 à 25°C.

**10.** Procédé selon l'une quelconque des revendications 6 à 9, dans lequel l'anticoagulant est un glycosaminoglycane.

**11.** Procédé selon la revendication 10, dans lequel l'anticoagulant est de l'héparine non fractionnée.

**12.** Procédé selon l'une quelconque des revendications 6 à 11, dans lequel l'anticoagulant est greffé sur au moins une surface de la membrane par contactant une solution aqueuse de l'anticoagulant avec la surface de la membrane.

**13.** Dispositif de filtration et/ou de diffusion comprenant une pluralité de membranes poreuses à fibres creuses selon l'une quelconque des revendications 1 à 5.

**14.** Dispositif de filtration et/ou de diffusion selon la revendication 13, dans lequel le dispositif est un hémodialyseur.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030021826 A1
- WO 2004056459 A1
- EP 2018069458 W
- US 5840190 A


### Non-patent literature cited in the description


- *Desalination and Water Treatment,* 2016, vol. 57 (7), 3218-3226